# FASCICULE DE BREVET EUROPEEN

(11) **EP 1 796 713 B1**
(45) Date de publication et mention de la délivrance du brevet: **09.03.2011**
(21) Numéro de dépôt: 05800554.7
(22) Date de dépôt: 09.09.2005
(51) Int. Cl.: A61K 38/17, C12N 5/10, A61P 37/00

(54) **UTILISATION DE LA PROTEINE GILZ POUR MODULER LA REPONSE IMMUNE SPECIFIQUE D'UN ANTIGENE**
VERWENDUNG VON GILZ-PROTEIN ZUR MODULATION EINER ANTIGENSPEZIFISCHEN IMMUNANTWORT
USE OF GILZ PROTEINTO MODULATE AN ANTIGEN-SPECIFIC IMMUNE RESPONSE

(30) Priorité: 10.09.2004 FR 0409620
(43) Date de publication de la demande: 20.06.2007
(73) Titulaire: Assistance Publique - Hôpitaux de Paris, 75004 Paris (FR)
(72) Inventeur: EMILIE, Dominique, F-75013 Paris (FR); COHEN, Nicolas, F-94160 Saint Mande (FR); LEMOINE, François, F-92210 Montrouge (FR)
(74) Mandataire: Leblois-Préhaud, Hélène Marthe Georgette
(86) Numéro de dépôt international: PCT/FR2005/002247
(87) Numéro de publication internationale: WO 2006/030118

(56) Documents cités:
- WO-A-03/054193
- ANONYMOUS: "Défis de la recherche en allergologie - Les 12 projets retenus dans le cadre du programme spécifique 2004" FONDATION RECHERCHE MÉDICALE, [Online] avril 2004 (2004-04), pages 1-5, XP002323452 Extrait de l'Internet: URL:http://www.frm.org/upload/presse/FRM-p rogallergies.pdf> [extrait le 2005-04-06]
- BERREBI DOMINIQUE ET AL: "Synthesis of glucocorticoid-induced leucine zipper (GILZ) by macrophages: an anti-inflammatory and immunosuppressive mechanism shared by glucocorticoids and IL-10." BLOOD. 15 JAN 2003, vol. 101, no. 2, 15 janvier 2003 (2003-01-15), pages 729-738, XP002323453 ISSN: 0006-4971

## Description

La présente invention est relative à l'utilisation de la protéine GILZ ou d'agent(s) modulant les effets de ladite protéine GILZ exprimée dans les cellules dendritiques en vue respectivement, d'induire des lymphocytes T régulateurs/suppresseurs pour générer une tolérance immunitaire, notamment dans le traitement des maladies autoimmunes, inflammatoires, des allergies, du rejet de greffe et de la maladie du greffon contre l'hôte, ou au contraire d'inhiber les lymphocytes T régulateurs/suppresseurs présents pour rompre un état de tolérance, notamment dans le traitement du cancer ou d'infections chroniques ou pour induire une réponse immune efficace contre un antigène vaccinal.

Les cellules dendritiques (CD) sont classiquement décrites comme étant les meilleures cellules présentatrices d'antigène aux lymphocytes T. En effet, les cellules dendritiques sont capables de présenter des antigènes peptidiques via les molécules du complexe majeur d'histocompatibilité de classe I et de classe II (CMH I et CMH II) respectivement aux lymphocytes T CD8⁺ et CD4⁺ et d'induire après activation une réponse immune effectrice. Des données plus récentes indiquent que sous l'effet de différents facteurs physiologiques et/ou d'agents pharmacologiques, les cellules dendritiques sont au contraire capables d'inhiber l'activation des lymphocytes T et ainsi d'induire un état de tolérance. La tolérance immune résulte de deux processus : l'induction de l'anergie ou de l'apoptose des cellules répondeuses et la génération de lymphocytes T possédant des fonctions suppressives (T régulateurs /suppresseurs).

Les lymphocytes T régulateurs/suppresseurs jouent un rôle majeur aussi bien physiologiquement que dans des situations pathologiques telles que les maladies auto-immunes, les allergies, le rejet de greffe, les infections chroniques et les proliférations malignes (Hisaeda et al., Nat. Med., 2004, 10, 29-. ; Lundgren et al., Infect. Immun., 2003, 71, 1755- ; Hasenkrug, Novartis Found. Symp., 2003, 252, 194-; Boyer et al., Blood, 2004, 103, 3428-3430 ; Woo et al., Cancer Res., 2001, 61, 4766- ; Sasada et al., Cancer, 2003, 98, 1089- ; Wolf et al., Clin. Cancer Res., 2003, 9, 606- ; Ichihara et al., Clin. Cancer Res., 2003, 9, 4404 ; Salomon et al., Immunity. 2000, 12, 431 ; Takahashi, Curr. Top. Med. Chem., 2003, 3, 693- ; Xu et al., J. Immunol ; 2003, 170, 394-).

Trois populations de lymphocytes T régulateurs/suppresseurs ont été décrites à ce jour : i) les cellules Th3 qui sont CD4⁺CD25⁻, produisent du *Transforming Growth Factor* beta (TGFß), de l'interleukine (IL)-10 et IL-4 tout en ayant une action suppressive par un mécanisme dépendant du TGFß (Weiner et al., Immunol. Rev., 2001, 182, 207-); *ii*) les cellules T régulatrices de type 1 (Tr1) qui représentent également une sous-population CD4⁺, produisent de l'IL-10 et du TGFß mais utilisent l'IL-10 pour leur différenciation et leur fonction suppressive (Groux, Transplantation, 2003, 75, 85-; Levings et al., J. Exp. Med., 2002, 196, 133) *iii*) les cellules CD4⁺CD25^{high} (Treg) dont l'action suppressive, initiée après une activation spécifique d'antigène via le récepteur des cellules T (TCR), s'exerce de manière indépendante de l'antigène en inhibant la production d'IL-2, et en favorisant l'arrêt du cycle cellulaire des cellules CD4⁺ et CD8⁺ par un mécanisme impliquant le contact cellulaire et partiellement le TGFß mais non l'IL-10 (Sakaguchi et al., Immunol. Rev., 2001, 182, 18- ; Shevach, Nat. Rev. Immunol., 2002, 2, 389-).

Bien que le phénotype, le profil d'expression cytokinique et les mécanismes suppresseurs des lymphocytes Th3, Tr1 et Treg aient été caractérisés, l'interaction des lymphocytes régulateurs/suppresseurs avec les cellules dendritiques et le rôle de cette interaction dans l'induction/inhibition des lymphocytes régulateurs/suppresseurs est mal connue. Il a été montré que le traitement des cellules dendritiques par les corticoïdes ou l'IL-10 induisait des lymphocytes T possédant des fonctions suppressives (Hackstein et al., précité ; Steinman et al., Ann. Rev. Immunol., 2003, 21, 685-711 ; Ashworth et al., Eur. J. Immunol., 2000, 30, 1233-1242 ; Muller et al., J. Invest. Dermatol., 2002, 119, 836-841 ; Steinbrink et al., Blood., 2002, 99, 2468-2476; Akbari et al., Nat. Immunol., 2001, 2, 725-731). Toutefois, les gènes capables d'induire ou au contraire d'inhiber des lymphocytes T régulateurs spécifiques de l'antigène, représentant des cibles thérapeutiques pour moduler les lymphocytes T régulateurs/suppresseurs n'ont pas été identifiés.

L'amélioration des connaissances chez l'homme concernant l'induction et l'inhibition des lymphocytes T régulateurs/suppresseurs permettrait d'envisager leur utilisation thérapeutique, soit pour favoriser ou induire un état de tolérance en vue du traitement de maladies auto-immunes, de maladies inflammatoires ou d'allergies ou du contrôle de la maladie du greffon contre l'hôte ou du rejet de greffe dans le cadre de transplantations allogéniques, soit au contraire pour induire une meilleure réponse immune contre un antigène tumoral, un antigène responsable d'infection chronique ou un antigène vaccinal, en les inhibant.

Le gène codant pour la protéine GILZ (*Glucocorticoid-Induced Leucine Zipper*) a été cloné à partir de lymphocytes T et de thymocytes murins cultivés en présence de glucocorticoïdes. La protéine GILZ murine et son homologue humain correspondent respectivement aux séquences SWISSPROT Q9Z2S7 et Q99576 et les ADNc codant lesdites protéines aux séquences GenBank AF024519 et AF228339. Dans ces cellules, GILZ inhibe la mort cellulaire (apoptose) induite par l'activation du récepteur T pour l'antigène (D'Adamio et al., Immunity, 1997, 7, 803). GILZ est également induit par les glucocorticoïdes dans des lymphocytes B et les macrophages humains (Berrebi et al., BLOOD, 2003, 101, 729-738). Dans les lymphocytes B, GILZ inhibe l'activation par le récepteur B pour l'antigène (Glynne et al., Immunol. Rev., 2000, 176, 216-). Dans les macrophages, GILZ agit au moins en partie en bloquant l'activation monocytaire induite par des extraits bactériens (LPS) ou des activateurs d'origine lymphocytaire T (CD40L, IFN-gamma) et inhibe la production de chimiokines pro-inflammatoires et des molécules co-stimulatrices CD80 et CD86 (Berrebi et al., précité). En outre, une dérégulation de l'expression de GILZ dans les macrophages est observée dans les processus inflammatoires liés aux réactions d'hypersensibilité retardée (répression de GILZ lors de la maladie de Crohn, ou de la tuberculose) et dans les tumeurs (persistance de l'expression de GILZ au cours des lymphomes de Burkitt). L'expression de GILZ peut également être induite dans d'autres types cellulaires, par différents stimulants : aldostérone et vasopressine dans des cellules tubulaires rénales murines (Robert-Nicoud et al., P.N.A.S., 2001, 98, 2712-), ou des cellules mésenchymateuses (Shi et al., EMBO Rep., 2003, 4, 374-).

La régulation de la production de GILZ inclut des sites de fixation du récepteur des glucocorticoïdes sur son promoteur, et la présence de cofacteurs de la famille de Sonic Hedgehog (Ingram et al., Oncogene, 2002, 21, 8196-) ou de la famille Forkhead (Asselin-Labat et al., Blood, 2004, 104, 215-223). Les effets de GILZ semblent s'exercer sur plusieurs voies d'activation cellulaire : la voie NF-kB (Riccardi et al., Adv. Exp. Med. Biol., 2001, 495, 31-), la voie AP-1 (Mittelstadt et Ashwell, J. Biol. Chem., 2001, 276, 29603-), la voie des MAP kinases (Ayroldi et al., Mol. Cell. Biol., 2002, 22, 7929-).

Il a notamment été montré que la protéine GILZ était un inhibiteur de la voie des MAP kinases capable de bloquer la transduction du signal médié par Raf/Ras, utile pour inhiber la prolifération cellulaire associée au cancer, aux maladies autoimmunes, aux pathologies inflammatoires et au rejet de greffe (Demande Internationale PCT WO 03/054193).

Les inventeurs ont mis en évidence de nouvelles propriétés de la protéine GILZ, liées à son expression dans des cellules autres que celles précitées, permettant d'envisager de nouvelles utilisations de la protéine GILZ.

Les inventeurs ont montré que l'expression de GILZ s'éteint au cours de la différenciation des précurseurs de cellules dendritiques (CD34+ ou monocytes) en cellules dendritiques immatures (iMDDC) et que l'activation des cellules dendritiques par du CD40L, qui permet leur maturation ne restaure pas cette production. Toutefois, l'induction de l'expression de GILZ dans les cellules dendritiques immatures (iMDDC) ou matures (MDDC) privilégie les fonctions tolérigènes des cellules dendritiques et inhibe leurs fonctions immunostimulantes. L'action tolérigène de GILZ s'exerce par l'induction de lymphocytes T suppresseurs spécifiques d'antigène et la production de GILZ par les cellules dendritiques est indispensable pour que ces cellules génèrent des lymphocytes T suppresseurs.

Ainsi, la modulation de la production ou de la fonction de la protéine GILZ dans les cellules dendritiques lors de la présentation d'antigène, par ses effets sur la génèse des lymphocytes T suppresseurs, permet de dicter la qualité de la réponse à cet antigène.

La mise en évidence de l'action de la protéine GILZ sur les cellules présentatrices d'antigène et de son effet sur l'induction de lymphocytes T régulateurs/suppresseurs, permet d'envisager l'utilisation de GILZ (protéine, fragment peptidique fonctionnel ou ADNc correspondant inclus dans un vecteur recombinant d'expression) ou d'un modulateur de GILZ (activateur ou inhibiteur), pour moduler la réponse immune spécifique d'un antigène. L'utilisation de GILZ et/ou d'un activateur de GILZ isolés ou exprimés dans des cellules dendritiques modifiées par l'ADNc ou la protéine correspondante, permet d'induire une tolérance à cet antigène, en vue du traitement de maladies auto-immunes, inflammatoires, d'allergies ou du contrôle du rejet de greffe et de la maladie du greffon contre l'hôte dans le cadre de transplantations allogéniques. L'utilisation d'un inhibiteur de GILZ, isolé ou exprimé dans des cellules dendritiques modifiées par l'ADNc ou la protéine correspondante, permet au contraire d'inhiber les lymphocytes T régulateurs/suppresseurs présents pour rompre un état de tolérance, notamment dans le traitement du cancer ou d'infections chroniques ou d'induire une réponse immune efficace contre un antigène tumoral ou un antigène d'un pathogène, dans le cadre d'une vaccination anti-tumorale ou anti-infectieuse.

La présente invention a en conséquence pour objet l'utilisation d'au moins: a) une protéine GILZ ou un vecteur recombinant d'expression de ladite protéine, isolés ou exprimés dans des cellules dendritiques modifiées, et b) un antigène sélectionné dans le groupe constitué par : un auto-antigène, un allergène, un antigène impliqué dans une maladie inflammatoire chronique ou un antigène de transplantation, pour la préparation d'un médicament destiné à la prévention et/ou au traitement des maladies auto-immunes, inflammatoires chroniques, des allergies, des rejets de greffe et de la maladie du greffon contre l'hôte.

### Définitions

- On entend par « protéine GILZ », une protéine GILZ de n'importe quel mammifère. La protéine GILZ humaine et son homologue murin correspondent respectivement aux séquences SWISSPROT Q99576 et Q9Z2S7 et les ADNc codant lesdites protéines aux séquences GenBank AF228339 et AF024519. La séquence de la protéine GILZ d'autres mammifères peut être déterminée par clonage de l'ADNc correspondant, à l'aide d'amorces choisies à partir des séquences humaines et murines, selon les techniques classiques de biologie moléculaire, connues de l'Homme du métier.
- On entend par « gène *GILZ* », le gène codant pour la protéine GILZ
- On entend par «protéine GILZ fonctionnelle ou fragment fonctionnel de la protéine GILZ », une protéine ou un peptide d'au moins 5 acides aminés consécutifs de GILZ qui, lorsqu'il est exprimé dans des cellules présentatrices d'antigène, est capable de générer des cellules T régulatrices/suppressives spécifiques de l'antigène. Des fragments de la protéine GILZ sont notamment décrits dans la Demande WO 03/054193. La fonctionnalité d'une protéine ou d'un peptide GILZ tel que défini ci-dessus peut-être évaluée par mise en évidence de l'inhibition de la prolifération des cellules T CD4⁺ et/ou T CD8⁺ spécifiques de l'antigène en présence de cellules présentatrices d'antigène autologues.
- On entend par « modulateur de GILZ », un activateur ou un inhibiteur de GILZ.
- On entend par « activateur de GILZ », un inducteur ou un activateur de l'expression du gène *GILZ* ou un activateur de la fonction de la protéine GILZ. Ledit inducteur ou activateur de l'expression du gène *GILZ* peut agir, soit directement en stimulant l'expression de GILZ, soit indirectement en bloquant l'action d'inhibiteurs de cette expression. Ledit activateur de la fonction de la protéine GILZ est notamment un co-facteur de GILZ, une substance agissant sur la phosphorylation, la glycosylation ou l'acylation de GILZ, ou bien une substance interférant avec la synthèse ou la fonction d'un co-facteur de GILZ.
- On entend par « inhibiteur de GILZ », un inhibiteur de l'expression du gène *GILZ* ou un inhibiteur de la fonction de la protéine GILZ ; ledit inhibiteur de l'expression du gène *GILZ* peut agir, soit directement, soit indirectement en bloquant l'action d'inducteurs ou d'activateurs de cette expression.
- On entend par « transcrit GILZ », l'ARNm codant pour la protéine GILZ.
- On entend par « cellules dendritiques modifiées » des cellules dendritiques, de préférence autologues, dans lesquelles une protéine GILZ exogène, un fragment fonctionnel d'au moins 5 acides aminés consécutifs de ladite protéine, un modulateur de GILZ ou un vecteur recombinant d'expression de ladite protéine, dudit fragment ou dudit modulateur de GILZ ont été introduits par tout moyen, connu en lui-même, permettant d'introduire une substance dans une cellule-cible.
- On entend par molécule permettant le ciblage et/ou le passage de la membrane plasmique des cellules dendritiques, toute molécule connue en elle-même, permettant d'introduire spécifiquement une substance dans les cellules dendritiques. A titre d'exemple non-limitatif on peut citer notamment, des peptides membranaires, des lipides, des ligands d'un récepteur membranaire ou d'un antigène de surface des cellules dendritiques, notamment des peptides, et des anticorps dirigés contre : DC-SIGN, CD40, DEC-205, la langérine, le récepteur pour le mannose ou les « scavengers receptor" (ou récepteurs d'épuration). Le récepteur membranaire DEC-205 est notamment décrit dans Bonifaz et al., J. Exp. Med., 2004, 199, 815-824.

L'invention décrit des protéines GILZ modifiées, notamment les variants de GILZ et les fragments d'au moins cinq acides aminés consécutifs desdites protéines correspondant à une protéine ou à un peptide GILZ fonctionnels tels que défini ci-dessus. Les modifications qui sont introduites dans la protéine ou le peptide GILZ par les techniques classiques connues de l'Homme du métier incluent de façon non limitative : mutation (insertion, délétion, substitution) d'au moins un acide aminé dans la séquence de GILZ (obtention de variants de GILZ), addition d'une séquence de fusion (obtention d'une protéine de fusion), substitution de résidus d'acides aminés par des résidus d'acides aminés non-naturels (acides aminés D ou analogues d'acides aminés), modification de la liaison peptidique, cyclisation, addition de groupements chimiques au niveau des chaînes latérales des acides aminés, couplage à une molécule d'intérêt, notamment une molécule permettant le ciblage et/ou le passage de la membrane plasmique des cellules dendritiques telle que définie ci-dessus ; ledit couplage est réalisé par l'intermédiaire d'une liaison non-covalente ou covalente telle qu'une liaison peptidique, notamment lorsque la protéine GILZ, un fragment de ladite protéine ou un modulateur de GILZ, de nature protéique, sont utilisés avec un ligand peptidique d'un récepteur membranaire ou d'un antigène de surface des cellules dendritiques (obtention d'une protéine ou d'un peptide GILZ chimériques).

Les modulateurs de GILZ comprennent notamment : les hormones gluco- ou minéralo-corticoïdes (ou leur dérivés) ; les hormones sexuelles (oestrogène, progestérone, androgène, ou leurs dérivés) ; les cytokines comme l'IL-10, l'IL-4, l'IL-13, le TGF beta ou des dérivés agissant sur leur récepteur ; la vitamine D et ses analogues ; les chimiokines naturelles et leurs variants, ligands de récepteurs tels que le récepteur CCR5 ; des protéines recombinantes, dérivées par exemple de CTLA4 ou de LAG-3 ; des immunoglobulines, polyclonales non spécifiques ou monoclonales ; HLA-G et ses formes solubles, naturelles ou recombinantes ; des substances agissant sur le récepteur pour les fragments Fc des immunoglobulines ; des substances interférant avec la fonction des Toll-like recepteurs (TLR), de CD36, de CD46, des récepteurs du complément, des « scavenger receptors » (ou récepteurs d'épuration, comme Lox-1 et MARCO), du récepteur au mannose, de DC-SIGN, et du récepteur à la phosphatidyl sérine. Il peut s'agir également de substances interférant avec la production ou la fonction de médiateurs intracellulaires, et en particulier les kinases akt, PI3K, et les phosphatases PTEN, SHP et SHIP, les protéines de la famille Forkhead, de la famille Smad, de la famille STAT (et en particulier STAT3). Il peut encore s'agir de drogues immunosuppressives comme la ciclosporine, le KF506, la rapamycine, le méthotrexate, le mycophénolate, l'azathioprine. Il peut s'agir de substances dérivées d'agents infectieux, en particulier de *Plasmodium falciparum, Leishmania, Candida albicans, Klebsiella pneumoniae, Toxoplasma gondii,* de *Trypanosoma cruzi, Mycobacterium tuberculosis, Porphyromonas gingivalis,* de protéines virales issues du VIH (comme vpr, gp41, gp120 et gp160), des virus de l'hépatite B ou de l'hépatite C, des herpès virus et des poxvirus.

L'antigène qui est utilisé est notamment sous forme d'une préparation antigénique, préparée notamment à partir de cellules, d'un antigène isolé (protéine, peptide ou dérivé ou autre) ou bien d'un vecteur recombinant d'expression dudit antigène.

L'antigène qui est utilisé pour induire une tolérance immune est choisi parmi les antigènes qui ont été identifiés comme responsables de la pathologie à traiter, à savoir de façon non-limitative :
- auto-antigènes impliqués dans des maladies auto-immunes telles que : thyroïdite, diabète, sclérose en plaque, neuropathie périphérique, maladie coeliaque, syndrôme de Goodpasture, polymyosite et dermatomyosite, polychondrite atrophiante, syndrôme des anti-phospholipides, vascularite, gastrite auto-immune, anémie hémolytique auto-immune, purpura thrombopénique auto-immun, hépatite auto-immune, phemphigus et pemphygoides, vitiligo, myasthénie, hémophilie, oedème angioneurotique auto-immun. Il s'agit notamment des auto-antigènes suivants : membrane basale, récepteurs de la TSH, de l'acétylcholine, beta2-glycoprotéine 1, C1-inactivateur, desmosomes et hémidesmosomes, facteur intrinsèque, gangliosides, insuline et pro-insuline, glutamate decarboxylase (GAD), *islet antigen 2* (IA2), *myelin-associated glycoprotein* (MAG) et *myelin basic protein,* tyrosinase, prothrombine, canaux calciques ou potassiques, thyroperoxydase, thyréoglobuline et gliadine.
- allergènes : pollen (pollens d'herbes et de céréales (*Phleum pratense, Zea mays, Secale cereale, Avena sativa, Anthoxanthum odotarum, Poa pratensis, Phragmites australis, Triticum sativum, Lolium perenne*))*,* venin d'hyménoptère (Api m1 et m2 : Phospholipase A2 et hyaluronidase d'*Apis mellifera ;* Ves v 1, *ves m 1, Pol a 1, Ves v* 2, Ves m 2, *Pol a* 2 (Phospholipase A1 et hyaluronidase de*vespula vulgaris, ger manica ou yellow jacket),* acariens (*Der p 1, Der p 2, Der f 1*), poil de chat (*Fel d* 1), arachide (*Ara h* 1,2 et 3), agent infectieux comme *Aspergilles fumigatus,* bouleau, olivier, farine, latex (*Hev b* 1 à 11), morue (*gad c* 1), crustacés et crevettes (*Pen a* 1), oeuf, lait de vache, et les protéines recombinantes correspondant à ces allergènes.
- antigènes impliqués dans des maladies inflammatoires chroniques telles que : psoriasis, polyarthrite rhumatoide et maladies inflammatoires du tube digestif.
- antigènes de transplantation impliqués dans le rejet de greffe et la maladie du greffon contre l'hôte tels que notamment les antigènes du complexe majeur d'histocompatibilité (CMH ou HLA chez l'homme) et les antigènes mineurs d'histocompatibilité.

L'antigène qui est utilisé pour induire une immunostimulation est choisi parmi n'importe quel antigène d'intérêt vaccinal. Il s'agit en particulier d'un antigène tumoral ou un antigène d'un un agent pathogène responsable d'infection aigue ou chronique, connu en lui-même. De façon non-limitative, on peut citer en particulier les antigènes de mélanome (Melan-A, Martl, tyrosinase), de tumeur épithéliale (Her2neu), de virus (VIH, VIS, virus des hépatites B et C), de *Plasmodium,* de mycobactéries.

Les vecteurs dans lesquels on peut insérer une séquence d'intérêt dans une cassette d'expression contenant les éléments régulateurs de transcription et de traduction appropriés sont connus de l'Homme du métier. Ces vecteurs sont construits par les méthodes classiques d'ADN recombinant et de génie génétique, qui sont connues en elles-mêmes. De nombreux vecteurs dans lesquels on peut insérer une molécule d'acide nucléique d'intérêt afin de l'introduire et de la maintenir dans une cellule hôte eucaryote sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extrachromosomique ou bien intégration dans le matériel chromosomique de l'hôte). On peut utiliser entre autres les acides nucléiques nus (ADN, ARN, linéaire ou circulaire), notamment les plasmides, et les vecteurs viraux tels que les adénovirus, les rétrovirus, les lentivirus et les AAV (Adeno-Associated-Virus), les poxvirus, et en particulier les canarypox, les herpès virus et le virus West-Nile.

Une utilisation, dans laquelle a) est un inhibiteur de GILZ ou un vecteur recombinant d'expression du précédent, isolé ou exprimé dans des cellules dendritiques modifiées, et ledit antigène défini en b) est un antigène d'intérêt vaccinal tel qu'un antigène tumoral ou un antigène d'un microorganisme pathogène, tels que définis ci-dessus, est également décrite.

On obtient ainsi un vaccin destiné à la prévention et/ou au traitement des pathologies tumorales et des infections, qui est particulièrement efficace dans la mesure où la réponse contre l'antigène vaccinal est potentialisée du fait des propriétés immunostimulantes dudit inhibiteur de GILZ, qui sont spécifiques de l'antigène vaccinal. En effet, ledit inhibiteur induit une réponse immune efficace contre l'antigène en inhibant la production ou la fonction de lymphocytes T régulateurs/suppresseurs spécifiques de l'antigène.

Une réponse immune efficace, ne générant pas de lymphocytes T suppresseurs ou inhibant leur fonction, peut être induite en thérapie vaccinale, préventive ou curative, afin d'améliorer l'immunogénicité de préparations antigéniques, par exemple pour les vaccinations anti-infectieuses déjà existantes (... hépatites B) ou à identifier (hépatites C, VIH). Cette possibilité d'accroître l'immunogénicité vaccinale est particulièrement intéressante en cas de faible immunogénicité de la préparation vaccinale, ou en cas de déficit immunitaire du sujet à vacciner (déficit immunitaire congénital, insuffisance rénale chronique, sujet âgé, infection par le VIH, paludisme, rougeole, ...).

L'amélioration de l'immunogénicité est également un objectif important dans le cadre de vaccinations anti-tumorales, en association avec des approches de thérapie cellulaire (vaccination par des cellules dendritiques présentant des antigènes tumoraux, génération ex-vivo de lymphocytes cytotoxiques anti-tumoraux) ou de vaccination par injection d'extraits tumoraux ou d'antigènes tumoraux caractérisés.

Une autre application porte sur l'activation directe du système immunitaire du sujet dans le cas où sa réponse spontanée contre un ou des antigènes est insuffisante, que cette insuffisance de réponse s'explique par un excès de lymphocytes T suppresseurs ou non. Par exemple, dans le cas d'une réponse anti-tumorale, d'une infection chronique comme l'hépatite virale chronique, l'infection par le VIH, les infections par herpes virus, la lèpre, la leishmaniose et le paludisme.

Selon une disposition avantageuse de ce mode de réalisation, ledit inhibiteur est un petit ARN interférent (siRNA) ciblant le transcrit GILZ ou un oligodésoxynucléotide antisens complémentaire dudit transcrit. De préférence, il s'agit d'un siRNA. A titre d'exemple non-limitatif, on peut citer le siRNA correspondant à la séquence SEQ ID NO : 1.

Des vecteurs particulièrement bien adaptés à l'expression stable des siRNA, sont notamment ceux décrits dans T.R. Brummelkamp et al., Science, 2002, 296, 550-553.

Selon une autre disposition avantageuse de ce mode de réalisation, ledit inhibiteur est un antagoniste de GILZ sélectionné dans le groupe constitué par : un antagoniste de la kinase akt ou un activateur de ladite kinase tel que la chimiokine RANTES.

Des antagonistes de la kinase akt sont notamment décrits dans Asselin - Labat et al., précité.

Par l'utilisation de l'invention.

On obtient un médicament destiné à la prévention et/ou au traitement des maladies auto-immunes, inflammatoires, des allergies, des rejets de greffe et de la maladie du greffon contre l'hôte.

Un tel médicament induit l'expression de GILZ *in vivo* dans les cellules dendritiques et génère des lymphocytes T régulateurs/suppresseurs spécifiques de l'antigène inducteur d'une tolérance immune spécifique de l'antigène. Ces lymphocytes T suppresseurs spécifiques d'antigène ont un intérêt thérapeutique au cours de pathologies caractérisées par un excès de réponse immune, et pour lesquelles l'antigène déclenchant est identifié. C'est le cas par exemple de la transplantation allogénique ou xénogénique d'organes ou de cellules souches, de maladies auto-immunes (thyroïdite, diabète, sclérose en plaque, neuropathie périphérique, maladie coeliaque), de maladies inflammatoires chroniques (psoriasis, polyarthrite rhumatoïde, maladie inflammatoire du tube digestif) ou d'allergie. Cet effet thérapeutique peut être préventif ou curatif. Par exemple, en transplantation d'organe, les lymphocytes T dérivés du receveur et suppresseurs d'une réponse contre le donneur peuvent prévenir ou guérir le rejet d'organe. Les lymphocytes T dérivés du donneur et suppresseurs d'une réponse contre le receveur peuvent prévenir ou guérir une réaction de greffon contre hôte. Dans le cas d'allergie, notamment aux venins d'hyménoptère, aux arachides, d'asthme ou d'autres maladies allergiques, des lymphocytes T suppresseurs de l'allergène peuvent prévenir les manifestations allergiques, la génération de tels lymphocytes T suppresseurs correspondant à une nouvelle approche de désensibilisation contre l'allergène. Cette stratégie peut-être adaptée en fonction de la découverte de nouveaux antigènes notamment de nouveaux auto-antigènes.

Selon une disposition avantageuse de ce mode de réalisation, ladite protéine GILZ définie en a) est la protéine humaine.

Un activateur de GILZ est également décrit, qui peut être un inducteur de l'expression du gène *GILZ* sélectionné dans le groupe constitué par : la dexaméthasone, l'IL-10, et le TGFß.

Selon une autre disposition avantageuse de ce mode de réalisation, ledit antigène défini en b) est sélectionné dans le groupe constitué par : *Phleum pratense, Dermatophagoides pteronyssinus (Der p 1, Der p* 2) et *farinae* (*Der f* 1), un antigène de latex (*Hev b* 1 à 11), la pro-insuline, MAG, la thyroperoxydase et la thyréoglobuline.

Selon un autre mode de réalisation avantageux de ladite utilisation, ladite molécule permettant le ciblage et/ou le passage de la membrane plasmique des cellules dendritiques est un ligand d'un récepteur ou d'un antigène membranaire sélectionné dans le groupe constitué par : DC-SIGN, CD40, DEC-205, la langérine, le récepteur pour le mannose et un récepteur d'épuration. Ledit ligand est notamment un peptide.

Selon un autre mode de réalisation avantageux de ladite utilisation, ladite molécule permettant le ciblage et/ou le passage de la membrane plasmique des cellules dendritiques est un anticorps dirigé contre un récepteur ou un antigène membranaire sélectionné dans le groupe constitué par : DC-SIGN, CD40, DEC-205, la langérine, le récepteur pour le mannose et un récepteur d'épuration.

Selon un autre mode de réalisation avantageux de ladite utilisation, ledit vecteur défini en a) comprend une cassette d'expression incluant un promoteur d'un gène exprimé de façon spécifique ou préférentielle dans les cellules dendritiques. De préférence, ledit promoteur est celui d'un gène codant pour une protéine sélectionnée dans le groupe constitué par: DC-SIGN, CD11c, une molécule du complexe majeur d'histocompatibilité et la langérine.

Selon un autre mode de réalisation avantageux de ladite utilisation, ledit vecteur d'expression défini en a) est un lentivirus.

Selon un autre mode de réalisation avantageux de ladite utilisation, ladite protéine GILZ ou ledit fragment sont sous la forme d'une protéine ou d'un peptide chimériques incluant la séquence dudit peptide permettant le ciblage et/ou le passage de la membrane plasmique des cellules dendritiques et/ou la séquence dudit antigène d'intérêt défini en b).

Selon un autre mode de réalisation avantageux de ladite utilisation, ledit vecteur recombinant d'expression défini en a) code pour une protéine GILZ ou un fragment de GILZ chimériques tels que définis ci-dessus.

Selon un autre mode de réalisation avantageux de ladite utilisation, ledit antigène défini en b) est chargé sur lesdites cellules dendritiques modifiées ou présenté par lesdites cellules définies en a).

La présente invention a également pour objet un médicament comprenant au moins des cellules dendritiques modifiées par une molécule sélectionnée dans le groupe constitué par: une protéine GILZ, un vecteur recombinant d'expression de ladite proteïne tel que défini ci dessus.

Selon un autre mode de réalisation avantageux dudit médicament, il comprend un antigène tel que défini ci-dessus, de préférence ledit antigène est chargé sur lesdites cellules dendritiques ou présenté par lesdites cellules.

La présente invention a également pour objet, l'utilisation des cellules dendritiques modifiées telles que définies ci-dessus, pour la préparation d'un médicament destiné à la prévention et/ou au traitement des maladies telles que définie ci-dessus.

Les cellules dendritiques sont des cellules dendritiques matures ou immatures, de préférence autologues, préparées selon les techniques de purification et de culture cellulaire connues de l'Homme du métier. Elles peuvent être isolées à partir des monocytes sanguins ou de cellules CD34⁺, dérivées de sang de cordon ombilical, de moelle osseuse ou du sang périphérique.

Pour obtenir de cellules dendritiques modifiées, lesdites substances sont introduites dans lesdites cellules en utilisant les méthodes classiques connues en elles-même : diffusion passive, électroporation, microinjection, association à toute(s) substance(s) permettant le ciblage et/ou le passage de la membrane plasmique des cellules dendritiques (peptides membranaires, transporteurs comme les nanotransporteurs, liposomes, nanoparticules, lipides, polymères cationiques, ligands notamment les peptides et les anticorps spécifiques des antigènes ou des récepteurs membranaires de surface tels que définis ci-dessus). En outre, on peut avantageusement combiner ces méthodes, par exemple en utilisant l'électroporation associée à des liposomes.

Avantageusement, lesdites substances sont couplés à une molécule permettant le ciblage et/ou le passage de la membrane plasmique des cellules dendritiques, notamment sous la forme d'une protéine ou d'un peptide chimérique ou d'un vecteur recombinant codant pour ladite protéine ou ledit peptide chimérique.

Selon un mode de réalisation avantageux dudit médicament, il comprend une protéine ou un peptide GILZ chimériques tels que définis à la ci-dessus.

Selon un autre mode de réalisation avantageux dudit médicament, il comprend un ou plusieurs vecteurs recombinants d'expression codant pour ladite protéine GILZ, et ledit antigène et/ou ladite molécule définis en b).

Selon un autre mode de réalisation avantageux dudit médicament, il comprend un vecteur recombinant codant pour une protéine ou un peptide GILZ chimériques tels que définis ci-dessus.

Conformément à l'invention les médicaments tel que définis ci-dessus peuvent être sous la forme d'une composition unique comprenant au moins une ou plusieurs substances définies en a) et un ou plusieurs antigène (s) et/ou molécule(s) de ciblage et/ou de passage de la membrane plasmique des cellules dendritiques définis en b). Alternativement, il peuvent être sous la forme d'une préparation combinée, dans laquelle la ou lesdites substances définies en a) et le ou lesdits antigène(s) et/ou molécule de ciblage et/ou de passage de la membrane plasmique des cellules dendritiques sont utilisés de façon séparée ou étalée dans le temps.

Les médicaments tels que définis dans la présente invention (protéine GILZ, ou vecteur recombinant isolé) peuvent être introduit *in vivo* dans les cellules-cibles (cellules dendritiques), soit par diffusion passive, soit en utilisant des méthodes physiques telles que l'électroporation ou la microinjection, soit en l'associant à toute(s) substance(s) permettant le ciblage et/ou le passage de la membrane plasmique des cellules dendritiques. Parmi les substances permettant le passage de la membrane plasmique, on peut citer notamment des peptides membranaires, des transporteurs comme les nanotransporteurs, des liposomes des nanoparticules, des lipides ou des polymères cationiques. Parmi les substances permettant le ciblage de la membrane plasmique des cellules dendritiques on peut citer notamment les ligands notamment les peptides et les anticorps spécifiques des antigènes ou des récepteurs membranaires de surface tels que définis ci-dessus. En outre, on peut avantageusement combiner ces méthodes, par exemple en utilisant l'électroporation associée à des liposomes.

Les médicaments tels que définis dans la présente invention peuvent notamment être administrés directement au niveau de leur site d'action, par exemple au sein d'une tumeur, d'un organe lymphoïde, ou au site d'administration d'un antigène vaccinal. Alternativement, ils peuvent être intégrés dans un liposome ou une nanoparticule, recouverts d'un ligand ou d'un anticorps monoclonal spécifique d'un antigène ou d'un récepteur membranaire de surface des cellules dendritiques tels que définis ci-dessus, de façon à être capturés par une cellule dendritique.

La posologie utile varie en fonction de l'affection à traiter, de la voie et du rythme d'administration, ainsi que de la nature et du poids de l'espèce à traiter (humaine ou animale). Le produit selon l'invention est utilisé par voie digestive (orale, sublinguale), parentérale, locale ou intra-site. Il peut se présenter sous la forme de comprimés, simples ou dragéifiés, de gélules, de granules, de sirop, de suppositoires, de préparations injectables, de pommades, de crèmes, de gels, d'aérosol, lesquels sont préparés selon les méthodes usuelles. Dans ces formes galéniques, le produit est incorporé à des excipients habituellement employés dans des compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La présente invention a également pour objet, un procédé d'induction de lymphocytes T suppresseurs/régulateurs *in vitro,* à partir d'un échantillon de tissu contenant des cellules T CD4⁺ et/ou CD8⁺, caractérisé en ce qu'il comprend au moins :
a1) la préparation de cellules dendritiques autologues exprimant la protéine GILZ, et, de façon simultanée ou séquentielle,
b1) l'incubation desdites cellules dendritiques définies en a1) avec un antigène tel que défini ci-dessus et ledit échantillon de tissu contenant lesdites cellules T CD4⁺ ou CD8⁺.

Les cellules dendritiques exprimant la protéine GILZ, sont des cellules dendritiques autologues matures ou immatures traitées par un activateur de GILZ tel que défini ci-dessus ou bien modifiées par un protéine GILZ, un fragment de cette protéine ou un vecteur d'expression des précédents telles que définies ci-dessus ; elles sont préparées comme précisé ci-dessus.

Le procédé selon l'invention est utile pour préparer des lymphocytes T suppresseurs/régulateurs *in vitro,* à partir d'un échantillon de tissu contenant des cellules T CD4⁺ et/ou CD8⁺, issu d'un patient à traiter ou d'un donneur, en cas de transplantation.

Ledit échantillon de tissu peut être du sang périphérique, une tumeur, de la moelle osseuse ou tout autre tissu, notamment un tissu à transplanter.

Ledit échantillon de tissu peut éventuellement être mis en culture, préalablement à l'étape b) d'incubation.

La présente invention a également pour objet, l'utilisation de lymphocytes T régulateurs/suppresseurs susceptibles d'être obtenus par le procédé d'induction tel que défini ci-dessus, pour la préparation d'un médicament destiné à la prévention et/ou au traitement des maladies autoimmunes, inflammatoires, des allergies, du rejet de greffe et de la maladie du greffon contre l'hôte.

La présente invention a également pour objet, un procédé d'inhibition des lymphocytes T suppresseurs/régulateurs *in vitro,* à partir d'un
échantillon de tissu contenant des cellules T CD4⁺ et/ou CD8⁺, caractérisé en ce qu'il comprend au moins :
a2) la préparation de cellules dendritiques autologues dans lesquelles la protéine GILZ est inhibée, et, de façon simultanée ou séquentielle,
b2) l'incubation desdites cellules dendritiques définies en a2) avec un antigène tel que défini ci-dessus et ledit échantillon de tissu contenant des cellules T CD4⁺ et/ou CD8⁺.

Les cellules dendritiques dans lesquelles l'expression de la protéine GILZ est inhibée, sont des cellules dendritiques autologues matures ou immatures traitées ou modifiées par un inhibiteur de GILZ tel que défini ci-dessus, notamment un petit ARN interférent (siRNA) ou un oligodésoxynucléotide antisens complémentaire du transcrit GILZ, un vecteur d'expression des précédents ou un antagoniste de GILZ ; elles sont préparées comme précisé ci-dessus.

Le procédé selon l'invention est utile pour inhiber la fonction de lymphocytes T suppresseurs/régulateurs *in vitro,* à partir d'un échantillon de tissu tel que défini ci-dessus, contenant lesdites cellules, issues d'un patient à traiter.

La présente invention a également pour objet, l'utilisation de lymphocytes T régulateurs/suppresseurs susceptibles d'être obtenus par le procédé d'inhibition tel que défini ci-dessus, pour la préparation d'un médicament destiné à la prévention et/ou au traitement des pathologies tumorales et des maladies infectieuses.

Selon un mode de mise en oeuvre avantageux desdits procédés, ils comprennent une étape supplémentaire de purification de la population de lymphocytes T CD4⁺ ou CD8⁺ résultant de l'étape b1) ou b2) et éventuellement de la sous-population de lymphocytes T CD4⁺ ou CD8⁺ correspondant aux lymphocytes T régulateurs/suppresseurs à l'aide d'anticorps dirigés contre des marqueurs de surface appropriés (anti-CD25 ou anti-GITR, par exemple).

Outre les dispositions qui précèdent, l'invention comprend encore d'autres dispositions, qui ressortiront de la description qui va suivre, qui se réfère à des exemples de mise en oeuvre du procédé objet de la présente invention, avec référence aux dessins annexés dans lesquels :

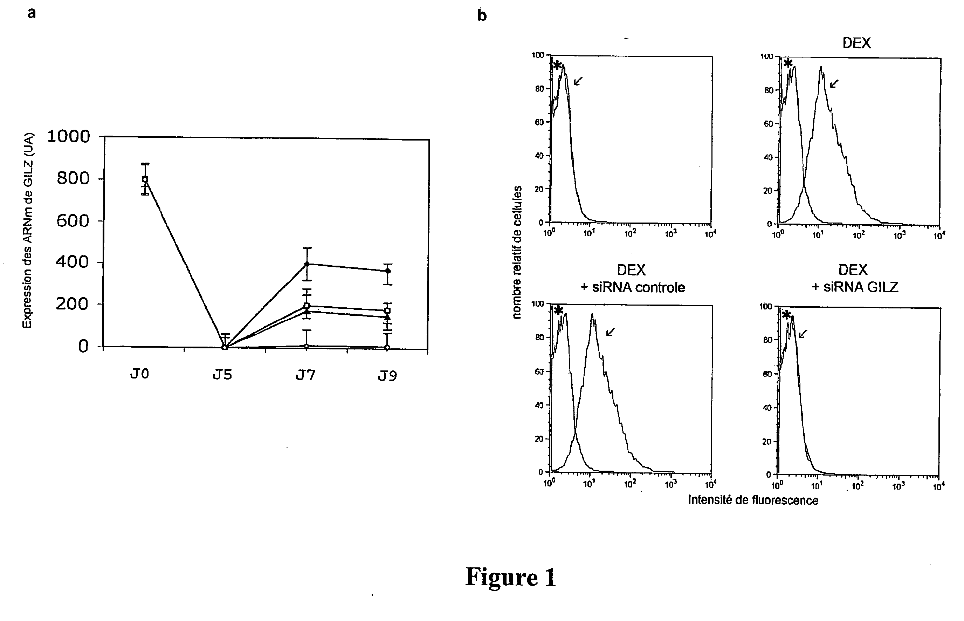

- la figure 1 illustre la production de GILZ par les cellules dendritiques (CD). a) Analyse par RT-PCR quantitative de l'expression du gène *GILZ* dans des cellules dendritiques traitées-par addition dans le milieu de culture- de dexaméthasone (Dex (◆), d'IL-10 (□) ou de TGFβ (▲) au cinquième jour de culture (J5) et de CD40 ligand (CD40L), au septième jour (J7). Les valeurs exprimées en unités arbitraires (AU) correspondent à la moyenne ± écart type de 3 à 6 expériences indépendantes. b) Analyse en cytométrie de flux de la production de la protéine GILZ dans les cellules dendritiques non-traitées (-) ou traitées par la dexaméthasone seule (DEX) ou avec petit ARN interférent (siRNA) contrôle (DEX + siRNA contrôle) ou ciblant GILZ (DEX+ siRNA GILZ). La courbe (↙) représente le marquage avec l'anticorps polyclonal anti-GILZ et l'anticorps secondaire (anticorps anti-isotypique) couplé à la phycoérythrine. La courbe (*) représente le marquage avec l'anticorps contrôle anti-isotypique seul.

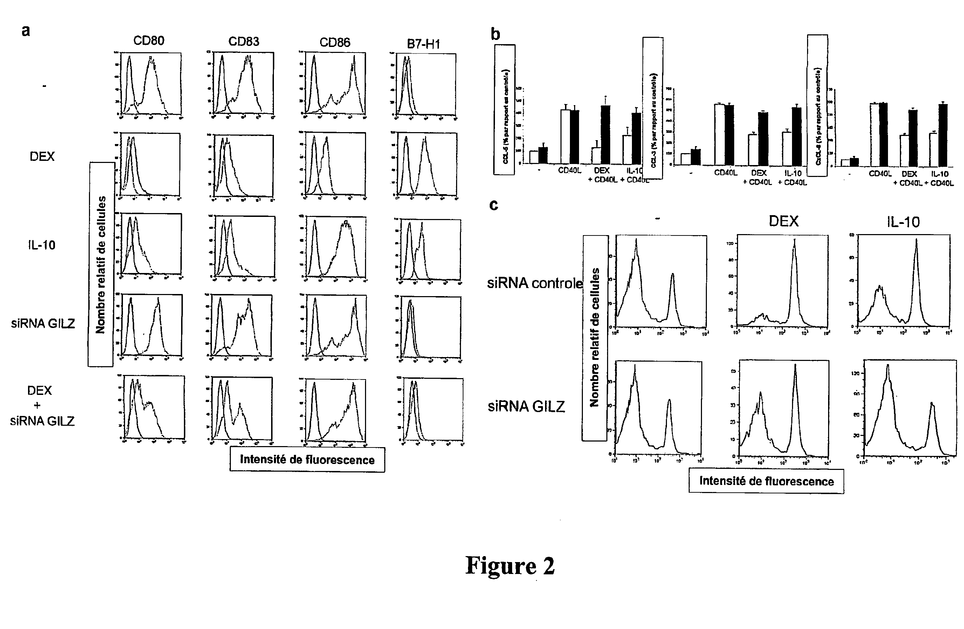

- la figure 2 illustre l'effet de l'inhibition de l'expression de GILZ dans les cellules dendritiques. Des cellules dendritiques matures dérivées de monocytes (MDDC) ont été traitées au cinquième jour de culture (J5) par un siRNA contrôle ou un siRNA ciblant GILZ (siRNA GILZ) seul, ou bien avec, soit de la dexaméthasone (DEX), soit de l'IL-10, ou non-traitées.
   a : Le CD40L a été ajouté à J7 et le phénotype des cellules dendritiques a été analysé à J9. Les résultats correspondent à une expérience représentative de 3 expériences indépendantes.
   b : Le CD40L a été ajouté à J7 et la production des chimiokines CCL3, CCL5 et CXCL8 a été analysée à J9. Les résultats correspondent à la moyenne ± écart type de 3 expériences indépendantes, exprimés sous la forme de pourcentage par rapport aux cellules dendritiques contrôle non-traitées. Les barres blanches correspondent aux cellules dendritiques transfectées par le siRNA contrôle alors que les barres noires correspondent aux cellules dendritiques transfectées par le siRNA-GILZ.
   c : L'antigène de rappel PPD (protéines standards purifiées de *Mycobacterium tuberculosis*) a été ajoutée à J7 et les lymphocytes T CD4+ autologues à J9. La prolifération des lymphocytes T CD4⁺ autologues a été analysée à J14 (une expérience représentative de 3 expériences indépendantes). Aucune prolifération n'est observée en absence de PPD.
- La figure 3 illustre les effets de GILZ sur les cellules dendritiques. Des cellules dendritiques matures (MDDC) ont été transduites par un vecteur vide (pcDNA3) ou un vecteur recombinant codant pour la protéine GILZ (pGILZ) puis stimulés par le CD40L. Le phénotype des cellules dendritiques a ensuite été analysé en cytométrie de flux. Les résultats correspondent à une expérience représentative de deux expériences indépendantes.
- La figure 4 illustre l'effet de l'expression de GILZ sur l'induction de lymphocytes T régulateurs.
   **a et b** : L'effet des lymphocytes T CD4⁺ stimulés par des cellules dendritiques chargées avec l'antigène de rappel PPD et traitées par la dexaméthasone et un siRNA contrôle (◆) ou anti-GILZ (○) a été testé sur une réponse proliférative anti-PPD (a) ou anti-CMV (b). Les valeurs exprimées en pourcentage par rapport aux lymphocytes T CD4+ contrôle (stimulés par de cellules dendritiques non-traitées) correspondent à la moyenne ± écart type de 3 expériences indépendantes**.**
   **c** : Les cellules présentatrices d'antigène circulantes isolées avant ou 48 heures après le début du traitement par un glucocorticoïde, ont été traitées avec un siRNA contrôle (◆) ou un anti-GILZ (○), chargées avec l'antigène PPD et utilisées pour activer des lymphocytes T CD4⁺ autologues. Ces lymphocytes ont été ajoutés en nombre croissant à des PBMC autologues stimulés par l'antigène PPD. Les résultats correspondent à une expérience représentative de deux expériences indépendantes.

### Exemple 1 : Mise en évidence de l'expression de GILZ dans les cellules dendritiques

### 1) Matériels et méthodes

### a) Purification et culture des cellules dendritiques

Des cellules dendritiques immatures dérivées de monocytes (iMDDCs) ont été préparées comme décrit dans Palucka et al., J. Immunol., 1998, 160, 4587-4595, à l'exclusion de modifications mineures. Brièvement, des monocytes humains ont été isolés à partir de la fraction de cellules mononuclées du sang périphérique par sélection négative, selon les recommandations du fabricant (DYNAL). Les monocytes (1 à 2.10⁵ cellules/ml) ont été cultivés pendant 7 jours en présence de GM-CSF et d'IL-4 (100 ng/ml et 50 ng/ml, respectivement, SCHERING-PLOUGH). La maturation des cellules dendritiques (MDDCs) a été induite en présence de CD40L (250 ng/ml ; IMMUNEX). La dexaméthasone (DEX, SIGMA), l'IL-10 recombinante humaine (rhIL-10, DNAX) et le TGFβ (R§D) ont été utilisés aux concentrations respectives suivantes : 10⁻⁷ M, 100 ng/ml et 20 ng/ml. Pour les expériences *in vivo,* les cellules présentatrices d'antigène (APC) ont été purifiées à l'aide du kit de sélection négative des monocytes, selon les recommandations du fabricant (DYNAL).

### b) Analyse de l'expression de GILZ

L'expression du gène *GILZ* humain a été analysé par RT-PCR en temps réel selon les recommandations du fabricant (Lightcycler ™, ROCHE), en utilisant le couple d'amorces décrit dans Berrebi et al., BLOOD, 2003, 101, 729-738 et le gène humain de la β actine comme contrôle interne.

L'expression de la protéine GILZ humaine a été analysée par cytométrie de flux intracellulaire à l'aide d'un anticorps polyclonal anti-GILZ décrit dans Asselin-Labat et al., (BLOOD, 2004, 104-215-) et d'un anticorps secondaire couplé à la phycoérythrine (BD PHARMINGEN).

### c) siRNAs et transfection

Les siRNAs sont synthétisés par MWG Biotec. Le siRNA ciblant GILZ (siRNA GILZ) présente la séquence 5'- AGUCCAGGAUUAUAGCCCCdTdT-3' (SEQ ID NO: 1). Le siRNA contrôle, constitué par des nucléotides choisis au hasard, correspond à un siRNA non-relevant ne présentant pas de spécificité connue (5'- ACG GGG GGC CCU UAA AAC AdTdT3', SEQ ID NO: 2).

La transfection des siRNAs est réalisée 48 heures avant la fin de la culture des IMDDC, à l'aide du kit Jetsi endo transfection kit ™ (Q-BIO GENE), selon les recommandations du fabricant.

### 2) Résultats

L'expression du gène *GILZ* au cours de la différenciation des cellules dendritiques dérivées des monocytes (MDDC) a été analysée par RT-PCR (figure 1 a).

Le gène *GILZ* est exprimé dans les monocytes fraîchement isolés mais son expression disparaît après 5 jours de culture en présence de GM-CSF et d'IL-4 et reste indétectable après l'induction de la maturation des cellules dendritiques par le CD40L (figure 1a).

L'addition de dexaméthasone (Dex), d'IL-10 ou de TGFβ au cinquième jour de culture stimule l'expression de GILZ qui est détecté avant et après le traitement par le CD40L (Figure 1 a).

Des résultats similaires ont été observés dans des cellules dendritiques dérivées de cellules CD34⁺.

La production de la protéine GILZ a été détectée par cytométrie de flux à l'aide d'un anticorps anti-GILZ, uniquement dans les cellules dendritiques traitées par la dexaméthasone mais pas dans les cellules contrôle non-traitées (figure 1b, panneau supérieur). L'expression de la protéine GILZ dans les cellules dendritiques traitées par la dexaméthasone est inhibée par un siRNA ciblant GILZ (figure 1b, panneau inférieur).

### Exemple 2 : Effet de l'expression de GILZ induite par la dexaméthasone ou l'IL-10 sur le phénotype, la production de chimiokines et l'activité stimulatrice des lymphocytes T des cellules dendritiques.

### 1) Matériels et méthodes

### a) Analyse du phénotype des cellules dendritiques

Le phénotype des cellules dendritiques a été analysé par cytométrie de flux à quatre couleurs, à l'aide des anticorps monoclonaux suivants : anti-CD83 couplé à phycoérythrine (CD83-PE, COULTER) ; anti-CD80 couplé à la phycoérythrine (CD80-PE, BD BIOSCIENCES) ; anti-B7-H1 (CLINISCIENCES) et anti-CD86 couplé à la phycoérythrine (CD86-PE, BD PHARMINGEN). L'anticorps anti-B7-H1 est détecté à l'aide d'un anticorps secondaire couplé à la phycoérythrine (BD PHARMINGEN).

### b) Co-culture de cellules dendritiques et de cellules T CD4+ et analyse de la prolifération des T CD4+

Des cellules dendritiques immatures dérivées de monocytes (iMDDC) ont été traitées au cinquième jour de culture (J5) par un siRNA contrôle ou un siRNA ciblant GILZ (siRNA GILZ) seul, ou bien avec, soit de la dexaméthasone (DEX), soit de l'IL-10, ou non-traitées, puis l'antigène de rappel PPD (protéines standards purifiées de *M. tuberculosis,* 1mg/ml, STATENS SERUM INSTITUTE) a été ajouté à J7. Les cellules dendritiques (0,5 x 10⁵) ainsi obtenues ont été lavées trois fois et co-cultivées avec 10⁵ cellules T CD4⁺ autologues fraîchement purifiées (TCD4 négative sélection kit™, DYNAL) dans des plaques de culture à 96 parts à fond rond, dans du milieu RPMI supplémenté avec 10 % de sérum humain de groupe AB. La prolifération des lymphocytes T CD4⁺ a été analysée au niveau individuel, au septième jour de co-culture (J14), à l'aide du colorant PKH26, comme décrit dans Rimaniol et al., Clin. Exp. Immunol., 2003, 132, 76-80.

### c) ELISA-cytokine

La production de chimiokines dans le surnageant de culture des cellules dendritiques a été analysée à l'aide de kits ELISA (R&D SYSTEMS) selon les instructions du fabricant.

### 2) Résultats

L'effet de GILZ sur le phénotype des cellules dendritiques et sur la production de chimiokines et la stimulation des lymphocytes T par ces cellules a été analysé.

La dexaméthasone et l'IL-10 modulent la maturation des cellules dendritiques ; elles inhibent l'expression de CD80, CD83 et CD86 et elles stimulent l'expression de B7-H1. Ces changements phénotypiques des cellules dendritiques induits par la dexaméthasone et l'IL-10 sont inhibés par le siRNA ciblant GILZ (Figure 2a).

L'activation des cellules dendritiques par le CD40L stimule la production des chimiokines CCL3, CCL5 et CXCL8. Cette induction est partiellement inhibée lorsque les cellules dendritiques sont traitées par la dexaméthasone ou l'IL-10 avant l'addition du CD40L. Le siRNA anti-GILZ renverse l'effet de la dexaméthasone et de l'IL-10 sur la production des chimiokines, alors que le siRNA contrôle est sans effet (Figure 2b).

Pour tester l'effet de GILZ sur la présentation de l'antigène, des cellules dendritiques ont été traitées à J5 avec un siRNA contrôle ou un siRNA anti-GILZ, seul ou en présence de dexaméthasone ou d'IL-10, ou non-traitées. Deux jours plus tard (J7), l'antigène de rappel PPD a été ajouté aux cultures de cellules dendritiques. Les cellules ont ensuite été lavées, mélangées à des lymphocytes T CD4⁺ autologues et la prolifération des lymphocytes T CD4⁺ a été analysée 7 jours après (J 14).

Le traitement des cellules dendritiques par la dexaméthasone diminue leur capacité de stimulation des lymphocytes T CD4⁺. Le siRNA anti-GILZ, seul ou le siRNA contrôle, seul ou en présence de dexaméthasone n'ont pas d'effet sur la réponse des lymphocytes T. En revanche, le siRNA anti-GILZ abolit l'effet de la dexaméthasone sur les cellules dendritiques (Figure 2c). Des résultats similaires sont observés avec les cellules traitées par l'IL-10 à la place de la dexaméthasone.

### Exemple 3 : Effet de GILZ sur les cellules dendritiques et l'induction de T régulateurs

### 1) Matériels et méthodes

### a) Vecteur recombinant d'expression de GILZ et transfection des cellules dendritiques dérivées de monocytes

Des cellules dendritiques ont été transfectées par un vecteur recombinant d'expression de GILZ dérivé de pcDNA3 (pGILZ ; Berrebi et al., précité) ou un vecteur vide (pcDNA3, IN VITROGEN), selon le protocole de transfection par nucléofection, à l'aide du système AMAXA^{™}.

### b) Induction de T régulateurs

Pour évaluer l'induction des T régulateurs (Treg), des cellules dendritiques immatures dérivées de monocytes traitées au cinquième jour de culture (J5) par un siRNA contrôle ou un siRNA ciblant GILZ (siRNA GILZ) seul, ou bien avec, soit de la dexaméthasone (DEX), soit de l'IL-10, ou non-traitées, puis traitées par le PPD à J7, ont été co-cultivées avec des cellules T CD4⁺, comme décrit à l'exemple 2. Les cellules T CD4⁺ ont été purifiées au septième jour de co-culture DC-T CD4⁺ et ajoutées en quantités croissantes à 10⁵ PBMC antologues stimulés par le PPD (1µg/ml, STATENS SERUM INSTITUTE) ou le cytomégalovirus (CMV, 25 µg/ml, BEHRING).

### 2) Résultats

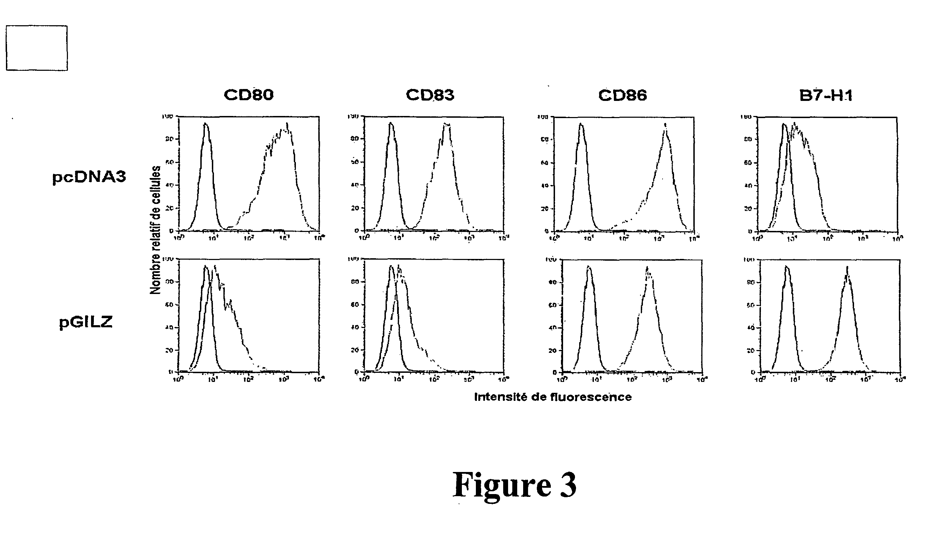

Les changements phénotypiques des cellules dendritiques induits par un vecteur d'expression recombinant codant pour la protéine GILZ (pcDNA3-GILZ) ont été analysés par cytométrie de flux, comme décrit à l'exemple 2, dans le but de déterminer si GILZ, à lui seul, était capable de reproduire les effets de la dexaméthasone et de l'IL-10 sur les cellules dendritiques. Les cellules dendritiques transduites par un vecteur d'expression de GILZ présentent un phénotype proche de celui des cellules dendritiques traitées par l'IL-10 ou la dexaméthasone, avec une expression faible de CD80, CD83 et CD86 et une expression forte de B7-H1 (figure 3).

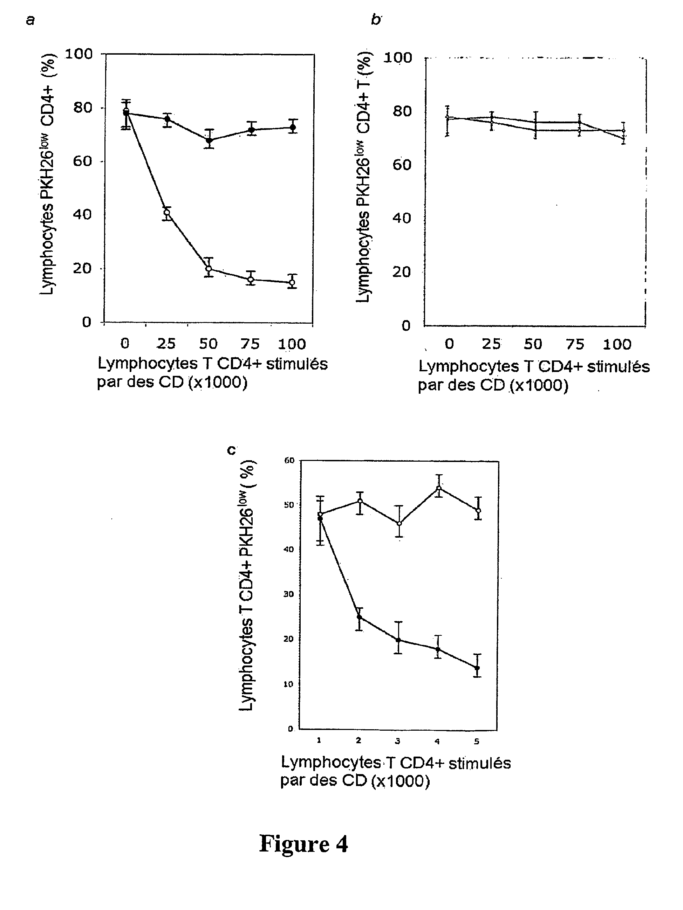

L'hypothèse selon laquelle les cellules dendritiques exprimant GILZ inhibent la réponse des lymphocytes T CD4⁺ en induisant des lymphocytes T régulateurs pendant la présentation de l'antigène a été vérifiée expérimentalement. Des cellules dendritiques ont été traitées avec un siRNA contrôle ou anti-GILZ, seul ou avec de la dexaméthasone, et avec du PPD (première culture). Après des lavages, les cellules dendritiques ont été utilisées pour stimuler des lymphocytes T CD4⁺ autologues. Les lymphocytes T CD4⁺ de cette seconde culture (lymphocytes T CD4⁺ sensibilisés) ont été purifiés et ajoutés en quantités croissantes à des PBMC autologues stimulés, soit par le PPD, soit par le CMV (troisième culture). Les lymphocytes T CD4⁺ sensibilisés par des cellules dendritiques non-traitées ou traitées par un siRNA contrôle n'ont pas d'effet sur la réponse proliférative de la troisième culture. Des lymphocytes T CD4+ sensibilisés par des cellules dendritiques traitées par la dexaméthasone et par un siRNA contrôle inhibent la réponse anti-PPD de la troisième culture, alors qu'ils n'ont pas d'effet sur la réponse anti-CMV. Ainsi, les cellules dendritiques traitées par la dexaméthasone induisent des lymphocytes T régulateurs spécifiques du PPD, pendant la présentation de l'antigène. La réponse de cette troisième culture est similaire lorsque les lymphocytes T CD4⁺ sont sensibilisés par des cellules dendritiques traitées par la dexaméthasone et par un siRNA anti-GILZ ou par des cellules dendritiques sensibilisées par un siRNA contrôle. Ces résultats indiquent que l'inhibition de la production de GILZ par les cellules dendritiques inhibe la capacité des cellules dendritiques traitées par la dexaméthasone à induire des lymphocytes T régulateurs (Figures 4a et 4b).

La capacité des cellules présentatrices d'antigène (APC) exprimant GILZ à induire des lymphocytes T régulateurs, *in vivo*, a également été testée.

L'administration de glucocorticoïde chez des individus augmente l'expression de GILZ par leurs cellules présentatrices d'antigène circulantes (535± 43 unités arbitraires (UA) d'ARNm GILZ deux jours après le début du traitement *versus* 151 ± 35 UA d'ARN m GILZ avant l'administration de glucocorticoïde, p<0,05). La capacité des cellules présentatrices d'antigène circulantes d'individus traités par des glucocorticoïdes à induire des lymphocytes T régulateurs a été testée comme décrit ci-dessus. Par comparaison avec des cellules présentatrices d'antigène prélevées avant le début du traitement, les cellules présentatrices d'antigène de patients traitées par les glucocorticoïdes, induisent des lymphocytes T CD4⁺ possédant des fonctions suppressives lors d'une réponse ultérieure anti-PPD, lorsqu'elles sont chargées avec la PPD. Ainsi, les glucocorticoïdes administrés chez l'Homme génèrent, *in vivo* comme *in vitro,* des cellules présentatrices d'antigène induisant des lymphocytes T régulateurs.

L'implication de GILZ dans ce phénomène a été testée en ajoutant une étape supplémentaire à l'expérience précédente : les cellules présentatrices d'antigène ont été incubées avec un siRNA contrôle ou un siRNA anti-GILZ, avant l'addition de PPD. L'induction de lymphocytes T régulateurs est complètement abolie lorsque les cellules présentatrices d'antigène sont cultivées en présence de siRNA anti-GILZ (figure 4c). En conséquence, le traitement des patients par les glucocorticoïdes induit la production de GILZ par leurs cellules présentatrices d'antigène, et cette expression de GILZ *in vivo* est indispensable pour induire des lymphocytes T régulateurs lors de la présentation de l'antigène.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Utilisation d'au moins: a) une protéine GILZ ou un vecteur recombinant d'expression de ladite protéine, isolés ou exprimés dans des cellules dendritiques modifiées, et b) un antigène sélectionné dans le groupe constitué par : un auto-antigène, un allergène, un antigène impliqué dans une maladie inflammatoire chronique ou un antigène de transplantation, pour la préparation d'un médicament destiné à la prévention et/ou au traitement des maladies auto-immunes, inflammatoires chroniques, des allergies, des rejets de greffe et de la maladie du greffon contre l'hôte.

2. Utilisation selon la revendication 1, **caractérisée en ce que** ladite protéine GILZ est la protéine humaine.

3. Utilisation selon la revendication 1 ou la revendication 2, **caractérisée en ce que** ledit antigène défini en b) est sélectionné dans le groupe constitué par : *Phleum pratense, Dermatophagoides pteronyssimus* et *farinae,* un antigène de latex tel que *Hev b* 1 à 11, la pro-insuline, MAG, la thyroperoxydase et la thyréoglobuline.

4. Utilisation selon l'une quelconque des revendications 1 à 3, **caractérisée en ce que** ladite protéine GILZ ou le vecteur recombinant d'expression de ladite protéine, isolés, sont associés à une molécule permettant le ciblage et/ou le passage de la membrane plasmique des cellules dendritiques, sélectionnée dans le groupe constitué par des peptides membranaires, des lipides, des ligands d'un récepteur membranaire ou d'un antigène de surface des cellules dendritiques et des anticorps dirigés contre : DC- SIGN, CD40, DEC-205, la langérine, le récepteur pour le mannose ou les récepteurs d'épuration.

5. Utilisation selon la revendication 4, **caractérisée en ce que** ledit récepteur membranaire ou antigène de surface des cellules dendritiques est sélectionné dans le groupe constitué par : DC-SIGN, CD40, DEC-205, la langérine, le récepteur pour le mannose et un récepteur d'épuration.

6. Utilisation selon l'une quelconque des revendications 1 à 5, **caractérisée en ce que** ledit vecteur comprend une cassette d'expression incluant un promoteur d'un gène exprimé de façon spécifique ou préférentielle dans les cellules dendritiques.

7. Utilisation selon la revendication 6, **caractérisée en ce que** ledit promoteur est celui d'un gène codant pour une protéine sélectionnée dans le groupe constitué par : DC-SIGN, CD11c, une molécule du complexe majeur d'histocompatibilité et la langérine.

8. Utilisation selon l'une quelconque des revendications 1 à 7, **caractérisée en ce que** ledit vecteur d'expression défini en a) est un lentivirus.

9. Utilisation selon l'une quelconque des revendications 1 à 8, **caractérisée en ce que** ladite protéine GILZ est sous la forme d'une protéine chimérique incluant la séquence dudit peptide permettant le ciblage et/ou le passage de la membrane plasmique des cellules dendritiques et/ou la séquence dudit antigène défini en b).

10. Utilisation selon l'une quelconque des revendications 1 à 9, **caractérisée en ce que** ledit antigène est chargé sur lesdites cellules dendritiques modifiées ou présenté par lesdites cellules.

11. Médicament comprenant au moins des cellules dendritiques modifiées par une molécule sélectionnée dans le groupe constitué par : une protéine GILZ et un vecteur recombinant d'expression de ladite protéine tels que définis à l'une quelconque des revendications 1, 2, 4 à 10.

12. Médicament selon la revendication 11, **caractérisé en ce qu'**il comprend également un antigène tel que défini à l'une quelconque des revendications 1, 3, 9 et 10.

13. Utilisation de cellules dendritiques modifiées telles que définies à la revendication 11 ou 12, pour la préparation d'un médicament destiné à la prévention et/ou au traitement des maladies telles que définies à la revendication 1.

14. Médicament comprenant au moins : a) une protéine GILZ ou un vecteur recombinant d'expression de ladite protéine, et b) un antigène d'intérêt et/ou une molécule permettant le ciblage et/ ou le passage de la membrane plasmique des cellules dendritiques, tels que définis à l'une quelconque des revendications 1 à 9.

15. Médicament selon la revendication 14, **caractérisé en ce qu'**il comprend un ou plusieurs vecteurs recombinants d'expression codant pour ladite protéine GILZ, ledit antigène et/ou ladite molécule permettant le ciblage et/ ou le passage de la membrane plasmique des cellules dendritiques, sélectionnée dans le groupe constitué par des peptides membranaires, des lipides, des ligands d'un récepteur membranaire ou d'un antigène de surface des cellules dendritiques et des anticorps dirigés contre : DC- SIGN, CD40, DEC-205, la langérine, le récepteur pour le mannose ou les récepteurs d'épuration.

16. Procédé d'induction de lymphocytes T suppresseurs/régulateurs *in vitro,* à partir d'un échantillon de tissu contenant des cellules T CD4⁺ et/ou CD8⁺, **caractérisé en ce qu'**il comprend au moins :
al) la préparation de cellules dendritiques autologues exprimant la protéine GILZ), et, de façon simultanée ou séquentielle,
b1) l'incubation desdites cellules dendritiques obtenues en a1) avec un antigène tel que défini à la revendication 1, 3, 9 ou 10, et ledit échantillon de tissu contenant lesdites cellules T CD4⁺ ou CD8⁺.

## Claims

1. Use of at least: a) a GILZ protein or a recombinant expression vector of said protein, isolated or expressed in modified dendritic cells, and b) an antigen selected from the group consisting of an autoantigen, an allergen, an antigen implicated in a chronic inflammatory disease or a transplantation antigen, for the preparation of a medicament for preventing and/or treating chronic inflammatory or autoimmune diseases, allergies, graft rejections and graft-versus-host disease.

2. Use according to Claim 1, **characterized in that** said GILZ protein is the human protein.

3. Use according to Claim 1 or Claim 2, **characterized in that** said antigen defined in b) is selected from the group consisting of *Phleum pratense, Dermatophagoides pteronyssinus* and *farinae,* a latex antigen such as Hev b 1 to 11, the proinsulin, MAG, the thyroperoxidase and the thyreoglobulin.

4. Use according to any one of Claims 1 to 3, **characterized in that** said GILZ protein or the recombinant expression vector of said protein, isolated, is associated with a molecule for targeting and/or passing through the plasma membrane of dendritic cells, said molecule being selected from the group consisting of membrane peptides, lipids, ligands of a membrane receptor or of a surface antigen of dendritic cells, and antibodies directed against DC-SIGN, CD40, DEC-205, the langerin, the mannose receptor or the scavenger receptors.

5. Use according to Claim 4, **characterized in that** said membrane receptor or surface antigen of dendritic cells is selected from the group consisting of DC-SIGN, CD40, DEC-205, the langerin, the mannose receptor and a scavenger receptor.

6. Use according to any one of Claims 1 to 5, **characterized in that** said vector comprises an expression cassette including a promoter of a gene expressed specifically or preferentially in dendritic cells.

7. Use according to Claim 6, **characterized in that** said promoter is that of a gene coding for a protein selected from the group consisting of DC-SIGN, CD11c, a molecule of the major histocompatibility complex, and the langerin.

8. Use according to any one of Claims 1 to 7, **characterized in that** said expression vector defined in a) is a lentivirus.

9. Use according to any one of Claims 1 to 8, **characterized in that** said GILZ protein is in the form of a chimeric protein including the sequence of said peptide for targeting and/or passing through the plasma membrane of dendritic cells, and/or the sequence of said antigen defined in b).

10. Use according to any one of Claims 1 to 9, **characterized in that** said antigen is loaded on to said modified dendritic cells or presented by said cells.

11. Medicament comprising at least dendritic cells modified by a molecule selected from the group consisting of a GILZ protein and a recombinant expression vector of said protein, as defined in any one of Claims 1, 2 and 4 to 10.

12. Medicament according to Claim 11, **characterized in that** it also comprises an antigen as defined in any one of Claims 1, 3, 9 and 10.

13. Use of modified dendritic cells as defined in Claim 11 or 12 for the preparation of a medicament for preventing and/or treating the diseases as defined in Claim 1.

14. Medicament comprising at least: a) a GILZ protein or recombinant expression vector of said protein, and b) an antigen of interest and/or a molecule for targeting and/or passing through the plasma membrane of dendritic cells, as defined in any one of Claims 1 to 9.

15. Medicament according to Claim 14, **characterized in that** it comprises one or more recombinant expression vectors coding for said GILZ protein, said antigen and/or said molecule for targeting and/or passing through the plasma membrane of dendritic cells, said molecule being selected from the group consisting of membrane peptides, lipids, ligands of a membrane receptor or of a surface antigen of dendritic cells, and antibodies directed against DC-SIGN, CD40, DEC-205, the langerin, the mannose receptor or the scavenger receptors.

16. Method of inducing suppressor/regulatory T lymphocytes *in vitro* from a tissue sample containing CD4⁺ and/or CD8⁺ T cells, **characterized in that** it comprises at least:
a1) the preparation of autologous dendritic cells expressing the GILZ protein, and, simultaneously or sequentially,
b1) the incubation of said dendritic cells obtained in a1) with an antigen as defined in Claim 1, 3, 9 or 10 and with said tissue sample containing said CD4⁺ or CD8⁺ T cells.

## Patentansprüche

1. Verwendung mindestens: a) eines GILZ-Proteins oder eines Vektors zur rekombinanten Expression des Proteins, isoliert oder exprimiert, aus modifizierten dendritischen Zellen, und b) eines Antigens, ausgewählt aus der Gruppe bestehend aus: einem Autoantigen, einem Allergen, einem Antigen, das mit einer chronischen Entzündungserkrankung oder einem Transplantationsantigen in Verbindung steht, zur Herstellung eines Medikaments zur Verhinderung und/oder Behandlung von Autoimmunerkrankungen, chronischen Entzündungserkrankungen, Allergien, Transplantatabstoßungen und der graft-versus-host Erkrankung.

2. Verwendung nach Anspruch 1, **dadurch gekennzeichnet, dass** das GILZ-Protein ein humanes Protein ist.

3. Verwendung nach Anspruch 1 oder Anspruch 2, **dadurch gekennzeichnet, dass** das in b) definierte Antigen ausgewählt ist aus der Gruppe bestehend aus: *Phleum pratense, Dermatophagoides pteronyssinus* und *farinae,* einem Latexantigen wie *Hev b 1* bis *11*, Pro-Insulin, MAG, Thyroperoxidase und Threoglobulin.

4. Verwendung nach einem der Ansprüche 1 bis 3, **dadurch gekennzeichnet, dass** das GILZ-Protein oder der Vektor zur rekombinanten Expression des Proteins isoliert mit einem Molekül assoziiert sind, das die Markierung und/oder die Passage der Plasmamembran der dendritischen Zellen erlaubt, ausgewählt aus der Gruppe bestehend aus Membranpeptiden, Lipiden, Liganden eines Membranrezeptors oder einem Oberflächenantigen der dendritischen Zellen und Antikörpern, die gerichtet sind gegen: DC-SIGN, CD40, DEC-205, Langerin, den Mannoserezeptor oder die Scavenger-Rezeptoren.

5. Verwendung gemäß Anspruch 4, **dadurch gekennzeichnet, dass** der Membranrezeptor oder das Oberflächenantigen der dendritischen Zellen ausgewählt ist aus der Gruppe bestehend aus: DC-SIGN, CD40, DEC-205, Langerin, dem Mannoserezeptor und einem Scavenger-Rezeptoren.

6. Verwendung nach einem der Ansprüche 1 bis 5, **dadurch gekennzeichnet, dass** der Vektor eine Expressionskassette mit einem Promotor für ein Gen umfasst, das spezifisch oder preferentiell in den dendritischen Zellen exprimiert wird.

7. Verwendung gemäß Anspruch 6, **dadurch gekennzeichnet, dass** der Promotor jener eines Gens ist, das für ein Protein kodiert, das ausgewählt ist aus der Gruppe bestehend aus: DC-SIGN, CD11c, einem Molekül des Haupthistokompatibilitätskomplexes und Langerin.

8. Verwendung gemäß einem der Ansprüche 1 bis 7, **dadurch gekennzeichnet, dass** der unter a) definierte Expressionsvektor ein Lentivirus ist.

9. Verwendung gemäß einem der Ansprüche 1 bis 8, **dadurch gekennzeichnet, dass** das GILZ-Protein in der Form eines chimären Proteins mit der Sequenz des Peptids, das die Markierung und/oder die Passage der Plasmamembran der dendritischen Zellen erlaubt und/oder der Sequenz des in b) definierten Antigens, vorliegt.

10. Verwendung gemäß einem der Ansprüche 1 bis 9, **dadurch gekennzeichnet, dass** das Antigen auf den modifizierten dendritischen Zellen angeordnet ist oder von den Zellen präsentiert wird.

11. Medikament umfassend mindestens durch ein Molekül modifizierte dendritische Zellen, das ausgewählt ist aus der Gruppe bestehend aus: einem GILZ-Protein und einem Vektor zur rekombinanten Expression des Proteins, wie in einem der Ansprüche 1, 2, 4 bis 10 definiert.

12. Medikament gemäß Anspruch 11, **dadurch gekennzeichnet, dass** es weiterhin ein Antigen, wie in einem der Ansprüche 1, 3, 9 und 10 definiert, umfasst.

13. Verwendung von modifizierten dendritischen Zellen wie in Anspruch 11 oder 12 definiert, zur Herstellung eines Medikaments zur Verhinderung und/oder Behandlung von Erkrankungen, wie in Anspruch 1 definiert.

14. Medikament umfassend mindestens: a) ein GILZ-Protein oder einen Vektor zur rekombinanten Expression des Proteins, und b) ein Antigen von Interesse und/oder ein Molekül, das die Markierung und/oder die Passage der Plasmamembran der dendritischen Zellen erlaubt, wie in einem der Ansprüche 1 bis 9 definiert.

15. Medikament gemäß Anspruch 14, **dadurch gekennzeichnet, dass** es einen oder mehrere Vektoren zur rekombinanten Expression zur Kodierung des GILZ-Proteins, des Antigens und/oder des Moleküls, das die Markierung und/oder die Passage der Plasmamembran der dendritischen Zellen erlaubt, ausgewählt aus der Gruppe bestehend aus: Membranpeptiden, Lipiden, Liganden eines Membranrezeptors oder eines Oberflächenantigens der dendritsichen Zellen und Antikörper, die gerichtet sind gegen: DC-SIGN, CD40, DEC-205, Langerin, den Mannoserezeptor und die Scavenger-Rezeptoren, umfasst.

16. Verfahren zur Induktion von Suppressor T-Lymphozyten/Regulator T-Lymphozyten *in vitro,* ausgehend von einer Gewebeprobe, die CD4⁺ und/oder CD8⁺T-Zellen enthält, **dadurch gekennzeichnet, dass** es mindestens umfasst:
a1) die Herstellung von autologen dendritischen Zellen, die das GILZ-Protein exprimieren, und, gleichzeitig oder aufeinanderfolgend,
b1) die Inkubation der unter a1) erhaltenen dendritischen Zellen mit einem Antigen, wie es in Anspruch 1, 3, 9 oder 10 definiert ist, und der Gewebeprobe, die die CD4⁺ und/oder CD8⁺T-Zellen enthält.


### SEQUENCE LISTING

<110> ASSISTANCE PUBLIQUE-HOPITAUX DE PARIS
   EMILIE, Dominique
   COHEN, Nicolas
   LEMOINE, François
<120> Utilisation de la protéine GILZ exprimée dans les cellules dendritiques pour moduler la réponse immune spécifique d'un antigène
<130> 1020PCT23
<160> 2
<170> PatentIn version 3.1
<210> 1
   <211> 21
   <212> DNA
   <213> artificial sequence
<220>
   <223> brin sens siRNA GILZ
<400> 1
   aguccaggau uauagcccct t 21
<210> 2
   <211> 21
   <212> DNA
   <213> ARTIFICIAL SEQUENCE
<220>
   <223> brin sens siRNA controle
<400> 2
   acggggggcc cuuaaaacat t 21